# EUROPÄISCHE PATENTANMELDUNG

(11) **EP 0 959 066 A2**
(43) Veröffentlichungstag der Anmeldung: **24.11.1999**
(21) Anmeldenummer: 99109862.5
(22) Anmeldetag: 19.05.1999
(51) Int. Cl.: C07C 233/36, A61K 7/075

(54) **Verwendung von Amiden polymerisierter Fettsäuren als Verdickungsmittel**

(30) Priorität: 20.05.1998 DE 19822791
(71) Anmelder: BASF AKTIENGESELLSCHAFT, 67056 Ludwigshafen (DE)
(72) Erfinder: Oppenländer, Knut, 67061 Ludwigshafen (DE); Zirnstein, Michael, 69198 Schriesheim (DE); Tiefensee, Kristin, 67368 Westheim (DE); Oetter, Günter, 67227 Frankenthal (DE)
(74) Vertreter: Kinzebach, Werner, Dr.

(57) **Zusammenfassung**

Die vorliegende Erfindung betrifft die Verwendung von Amiden polymerisierter Fettsäuren als Verdickungsmittel.

Darüber hinaus betrifft die Erfindung wässrige Zusammensetzungen, welche diese Amide als Verdickungsmittel enthalten.

## Beschreibung

Die vorliegende Erfindung betrifft die Verwendung von Amiden polymerisierter Fettsäuren als Verdickungsmittel sowie wässrige Zusammensetzungen, welche diese Amide enthalten.

Verdickungsmittel werden in großem Umfang zur Erhöhung der Viskosität von wässrigen Zubereitungen eingesetzt, beispielsweise auf dem Gebiet der Pharmazie und der Kosmetik. Beispiele für häufig verwendete Verdickungsmittel sind Fettsäurepolyethylenglycolmonoester, Fettsäurepolyethylenglycoldiester, Fettsäurealkanolamide, oxethylierte Fettalkohole, ethoxylierte Glycerinfettsäureester, Celluloseether, Natriumalginat, Polyacrylsäuren sowie Neutralsalze.

Die Verwendung der bekannten Verdickungsmittel ist jedoch, je nach der zu verdickenden Zubereitung, mit Nachteilen verbunden. So kann die Verdickungswirkung und die Salzstabilität der Verdickungsmittel nicht zufriedenstellend, ihr Einsatz unerwünscht und ihre Einarbeitung in die zu verdickende Zubereitung erschwert sein.

Verdickungsmittel auf Basis von Dimerfettsäuren haben sich insbesondere auf dem Gebiet der Kosmetik als vorteilhaft erwiesen. So beschreibt die EP-A-229 400 mit Fettsäuren modifizierte Polyester aus Polyalkylenoxid und dimerisierten Fettsäuren und deren Verwendung zur Erhöhung der Viskosität in tensidhaltigen kosmetischen, pharmazeutischen und technischen Präparaten. Neben der gewünschten Verdickungswirkung besitzen diese Polyester bei Verwendung in Kombination mit Kochsalz eine verringerte Korrosionswirkung und bei Anwendung auf dem Haar wird ein zusätzlicher Konditioniereffekt erzielt.

Die EP-A-507 003 beschreibt protonierte oder quaternisierte Esteramine und Amidoamine von Dimerfettsäuren als Weichspüler in wässrigen Weichspülmitteln und als Reinigungs- oder Pflegekomponente in wässrigen Dusch- und Haarshampoos sowie wässrigen Haarkonditionierungsmitteln.

Die US-A-4,636,326 beschreibt Verdickungsmittelzusammensetzungen, welche neben einem wasserlöslichen, thermoplastischen, organischen Polymer einen wasserlöslichen Polyester aus Dimerfettsäuren und Polyethylenglykolen enthält. Die Zusammensetzung ist zur Verdickung von wässrigen Hydraulikflüssigkeiten und Flüssigkonzentraten zur Metallbearbeitung brauchbar.

Die WO 95/12650 beschreibt Polyester aus Dimerfettsäuren, Fettsäuren und oxethylierten Polyalkoholen als Verdickungsmittel für wässrige Personal-Care-Produkte.

Die US-A-4,795,581 beschreibt wässrige Zusammensetzungen, welche Amide aus Fettsäuren oder Dimerfettsäuren und höhermolekularen Polyalkylenglykoldiaminen enthalten. Die Zusammensetzungen sind zur Verdickung von wässrigen Hydraulikflüssigkeiten sowie von wässrigen Kosmetik- und Reinigungsformulierungen brauchbar.

Die DE-A-195 05 196 beschreibt niedrigviskose wässrige Konzentrate mit mindestens 40 Gew.-% Betaintensiden und Dicarbonsäuremonoamiden und/oder Dicarbonsäurediamiden. Geeignete Dicarbonsäuremonoamide oder -diamide sind Umsetzungsprodukte der Dimerfettsäure mit aliphatischen Aminen, wie insbesondere Diethanolamin und Dimethylaminopropylamin. Die Dicarbonsäuremonoamide oder -diamide dienen zur Herabsetzung der Viskosität der Konzentrate.

Überraschenderweise wurde nun gefunden, dass Amide polymerisierter Fettsäuren als Verdickungsmittel für tensidhaltige Zusammensetzungen, insbesondere für Zusammensetzungen, welche ein Alkyl- oder Alkenylpolyglycosid enthalten, brauchbar sind.

Gegenstand der vorliegenden Erfindung ist daher die Verwendung von Amiden polymerisierter Fettsäuren der Formel I: 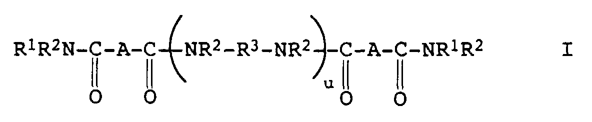 worin die Variablen unabhängig voneinander folgende Bedeutung haben:
- A: Rest einer dimerisierten Fettsäure mit 12 bis 110 C-Atomen (der nach Entfernung der Carboxylgruppen verbleibt;
- R¹: C₁-C₁₂-Alkyl- oder C₃-C₈-Cycloalkylrest, der gegebenenfalls 1 bis 6 Substituenten aufweisen kann, die unabhängig voneinander ausgewählt sind unter Hydroxy, C₁-C₄₋Alkoxy, das gegebenenfalls durch 1 oder 2 Hydroxy- oder C₁-C₄-Alkoxygruppen substituiert sein kann, Amino, C₁-C₄-Monoalkylamino und Di-C₁-C₄-alkylamino, wobei die Alkylgruppen der Aminogruppen unabhängig voneinander durch 1 oder 2 Hydroxygruppen substituiert sein können;
- R²: H oder C₁-C₄-Alkyl, das durch 1 oder 2 Hydroxygruppen substituiert sein kann;
- R³: C₂-C₁₂-Alkylen, das 1 bis 6 Substituenten aufweisen kann, die unabhängig voneinander ausgewählt sind unter Hydroxy, C₁-C₄-Alkoxy, das durch 1 oder 2 Hydroxygruppen substituiert sein kann, Amino, C₁-C₄-Monoalkylamino und Di-C₁-C₄-alkylamino, wobei die Alkylgruppen der Aminogruppen durch 1 oder 2 Hydroxygruppen substituiert sein können;
- u: 0 bis 20;
als Verdickungsmittel.

Die Alkylgruppen (auch in Gruppen wie Alkylamino, Alkoxy etc.) können geradkettig oder verzweigt sein. Beispiele sind Methyl, Ethyl, n-Propyl, 1-Methylethyl, n-Butyl, 1-Methylpropyl, 2-Methylpropyl, 1,1-Dimethylethyl, n-Pentyl, n-Hexyl, 2-Ethylhexyl, n-Dodecyl.

Bevorzugte Cycloalkylgruppen sind Cyclopentyl und Cyclohexyl.

R¹ steht vorzugsweise für einen C₁-C₆-Alkylrest, der mit 1 bis 6 Hydroxy- oder Aminogruppen substituiert sein kann.

R³ steht vorzugsweise für C₂-C₆-Alkylen, d. h. für (CH₂)₂₋₆, das gegebenenfalls einen oder zwei Hydroxy- oder Alkoxysubstituenten aufweisen kann.

u steht vorzugsweise für 0 bis 10, insbesondere für 0 bis 5 und besonders bevorzugt für 0.

Unter polymerisierten Fettsäuren sind gesättigte oder ungesättigte Fettsäuren mit 12 bis 110 C-Atomen, bevorzugt 24 bis 44 C-Atomen, besonders bevorzugt 32 bis 40 C-Atomen, zu verstehen, die durch Polymerisation einer oder verschiedener ungesättigten Fettsäuren hergestellt werden.

Die polymerisierbaren Fettsäuren sind einfach oder mehrfach ungesättigte Verbindungen mit einer Kohlenstoffkette von 6 bis 22 C-Atomen, bevorzugt 12 bis 22 C-Atomen, besonders bevorzugt 16 bis 20 C-Atomen, sowie Gemische dieser Fettsäuren, beispielsweise Ölsäure-Linolsäure-Gemische.

Die Polymerisation der Fettsäuren kann zu dimeren, trimeren, tetrameren und pentameren Strukturen führen. Bevorzugt sind dimere und trimere, insbesondere dimere Fettsäuren.

Die dimerisierten Derivate enthalten im wesentlichen lineare und cyclische Verbindungen, die ungesättigt oder hydriert sein können, bevorzugt aber hydriert sind.

Beispiele für ungesättigte Dimerfettsäurestrukturen sind:

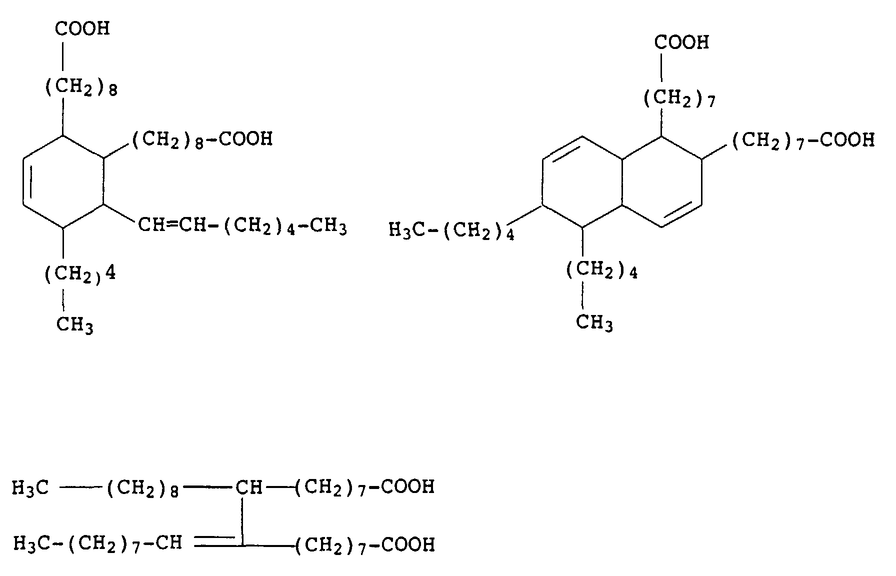

Als polymerisierte Fettsäuren kommen vorzugsweise die Produkte in Frage, die unter der Bezeichnung Pripol® (Fa. Unichema) oder Empol® (Fa. Henkel) im Handel erhältlich sind. Diese dimerisierten Öl-/Linolsäure Gemische enthalten vorwiegend lineare und cyclische Verbindungen. Daneben können diese Produkte auch noch Anteile von monomeren sowie von trimeren und höher kondensierten Fettsäuren enthalten.

Typische im Handel erhältliche dimere Fettsäuren haben etwa folgende Zusammensetzung:

| | |
|---|---|
| Monomere Säuren: | 0-15 Gew.-%, |
| dimere Säuren: | 50-99 Gew.-%, |
| tri- und höherpolymerisierte Säuren: | 1-35 Gew.-%, |

wobei der Gehalt je nach Herkunft der Monomeren, des Polymerisationsverfahrens sowie des Aufarbeitungsprozesses innerhalb dieser Grenzen schwanken kann.

Die polymerisierten Fettsäureamide werden hergestellt durch Kondensation mit einem Amin der Formel R¹R²NH und gegebenenfalls mit einem Amin der Formel HR²N-R³-NHR², wobei R¹ bis R³ die oben angegebenen Bedeutungen besitzen.

Als Amine kommen bevorzugt primäre hydrophile Amine in Frage (R² = H). Besonders bevorzugt sind Hydroxyamine mit 1, 2, 3, 4, 5 oder 6 Hydroxygruppen, wie Monoethanolamin, 1-Amino-2-propanol, 2-Amino-1-propanol, 3-Amino-1-propanol, Aminobutanole, wie 2-Amino-1-butanol, 4-Amino-1-butanol, 2-Amino-2-methyl-1-propanol, Aminopentanole, Aminohexanole und Aminocyclohexanole. Geeignete Hydroxyamine mit mehreren Hydroxygruppen sind beispielsweise 1-Amino-2,3-dihydroxypropan, 2-Amino-1,3-dihydroxypropan sowie Aminozucker, wie Aminosorbit (Glucamin), N-Methylglucamin, Glucosamin und Galactosamin.

Weitere geeignete Amine sind Alkoxyamine, Hydroxyalkoxyamine (R¹ = C₁-C₁₂-Alkyl, das durch C₁-C₄-Alkoxy oder Hydroxy-C₁-C₄-Alkyl substituiert ist),wie 1-Amino-2-methoxyethan, 1-Amino-2-ethoxyethan, 2-Amino-1-methoxypropan, 3-Amino-1-ethoxypropan, 2-(2-Aminoethoxy)ethanol und 3-(2-Methoxyethoxy)-1-propanamin.

Auch aliphatische primäre Amine sind geeignet, wie Methylamin, Ethylamin, 1-Propanamin, 2-Propanamin, 1-Butanamin, 2-Butanamin, Cyclopentanamin und Cyclohexanamin.

Geeignet sind auch hydrophile sekundäre Amine, wie Diethanolamin, N-Methylethanolamin, N-Ethylethanolamin, 3-(2-Hydroxyethylamin)-1-propanol, Diisopropanolamin und Di-(2-methoxyethyl)amin.

Auch primäre und sekundäre Diamine sind brauchbar (R¹ = C₁-C₁₂-Alkyl, das durch Amino, C₁-C₄-Monoalkylamino oder Di-C₁-C₄-alkylamino substituiert ist; R² = H, C₁-C₄-Alkyl), beispielsweise 1,2-Diaminoethan, 1,2-Diaminopropan, 1,3-Diaminopropan, 1,4-Diaminobutan und die Mono- und Dimethylderivate davon.

Gegenstand der vorliegenden Erfindung sind auch die Amide polymeriserter Fettsäuren der Formel I, worin R¹ den Rest eines Aminozuckers (nach Entfernung eines Wasserstoffatoms von der Aminogruppe) bedeutet und R², R³, A und n die oben angegebenen Bedeutungen besitzen. R¹ steht vorzugsweise für den Rest eines von Pentosen oder Hexosen abgeleiteten Aminozuckers, insbesondere für einen Aminosorbit-, Glucosamin- oder Galactosaminrest. R² steht vorzugsweise für H oder CH₃.

Die Herstellung der polymerisierten Fettsäureamide erfolgt nach an sich bekannten Methoden, beispielsweise durch Umsetzung der oben erwähnten Amine mit einer polymerisierten Fettsäure oder mit einem Ester einer polymerisierten Fettsäure, insbesondere dem Dimethylester (Amidierung). Die Amidierung kann unter üblichen Bedingungen ohne Katalysator oder unter Verwendung eines sauren oder basischen Katalysators durchgeführt werden. Geeignete saure Katalysatoren sind beispielsweise Säuren, wie Lewissäuren, z. B. Schwefelsäure, p-Toluolsulfonsäure, phosphorige Säure, hypophosphorige Säure, Phosphorsäure, Methansulfonsäure, Borsäure, Aluminiumchlorid, Bortrifluorid, Tetraethylorthotitanat, Zinndioxid, Zinnbutyldilaurat oder Gemische davon. Geeignete basische Katalysatoren sind beispielsweise Alkoholate, wie Natriummethylat oder Natriumethylat, Alkalimetallhydroxide, wie Kaliumhydroxid, Natriumhydroxid oder Lithiumhydroxid, Erdalkalimetalloxide, wie Magnesiumoxid oder Calciumoxid, Alkali- und Erdalkalimetallcarbonate, wie Natrium-, Kalium- und Calciumcarbonat, Phosphate, wie Kaliumphosphat, und komplexe Metallhydride, wie Natriumborhydrid.

Der Katalysator wird im Allgemeinen in Mengen von 0,05 bis 10 Gew.-%, vorzugsweise 0,1 bis 5 Gew.-%, bezogen auf die Gesamtmenge der Ausgangsstoffe, eingesetzt.

Die Umsetzung kann in einem geeigneten Lösungsmittel oder vorzugsweise lösungsmittelfrei durchgeführt werden. Bei Verwendung eines Lösungsmittels sind beispielsweise Kohlenwasserstoffe, wie Toluol oder Xylol, Acetonitril, N,N-Dimethylformamid, N,N-Dimethylacetamid, N-Methylpyrrolidon, Diethylenglykoldimethylether, Ethylenglykoldimethylether, Ethylencarbonat, Propylencarbonat etc. geeignet. Im Allgemeinen wird das Lösungsmittel während der Umsetzung oder nach beendeter Umsetzung abdestilliert.

Die Amidierung erfolgt bei Verwendung einer polymerisierten Fettsäure im Allgemeinen bei einem Druck im Bereich von 5 mbar bis Normaldruck und bei einer Temperatur im Bereich von 60 bis 220 °C, vorzugsweise 120 bis 180 °C. Bei Verwendung eines Esters einer polymerisierten Fettsäure erfolgt die Amidierung im Allgemeinen bei 30 bis 220 °C, vorzugsweise bei 60 bis 120 °C, und bei einem Druck im Bereich von 5 mbar bis Normaldruck. Die Reaktionszeiten liegen im Allgemeinen im Bereich von 2 bis 20 Stunden. Der Grad der Umsetzung lässt sich über die Menge an entferntem Reaktionswasser bzw. Reaktionsalkohol oder über die Bestimmung der Säurezahl und Aminzahl des Produktes verfolgen. Nicht umgesetztes Amin wird im Allgemeinen nach beendeter Umsetzung in üblicher Weise entfernt, beispielsweise im Vakuum und/oder Stickstoffstrom.

Die Edukte werden im Allgemeinen in equimolaren Mengen oder in einem Überschuss von bis zu etwa 5 bis 10 Mol-% eingesetzt. Es können jedoch auch größere Aminmengen verwendet werden, insbesondere bei Einsatz leicht zugänglicher Amine.

Bei Anwendung von Diaminen kann es zu einer Polykondensation kommen (u = 1 bis 20).

Alternativ kann man die Amide der polymerisierten Fettsäuren auch durch Umsetzung aus einem Fettsäurechlorid mit der Aminkomponente nach an sich bekannten Methoden erhalten.

Gegenstand der vorliegenden Erfindung sind auch wässrige Zusammensetzungen, die wenigstens ein Amid einer polymerisierten Fettsäure der wie oben definierten Formel I und wenigstens ein Tensid enthalten.

Gemäß einer Ausführungsform enthalten die Zusammensetzungen kein Betaintensid und insbesondere kein Betaintensid der Formel 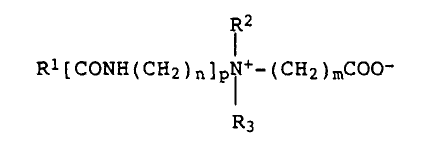 in der R¹ für einen aliphatischen Alkylrest mit 8 bis 22 C-Atomen, R² und R³ unabhängig voneinander für einen Alkyl- und/oder Hydroxyalkylrest mit 1 bis 4 C-Atomen, n und m unabhängig voneinander für Zahlen im Bereich von 1 bis 5 und p für 0 oder 1 steht.

Gegenstand der Erfindung sind weiterhin wässrige Zusammensetzungen, die enthalten:
a) wenigstens ein Alkyl- oder Alkenylpolyglycosid, insbesondere ein C₈-C₁₈-Alkyl- oder C₈-C₁₆-Alkenylpolyglykosid,
b) wenigstens ein Amid einer polymerisierten Fettsäure der wie oben definierten Formel I,
c) gegebenenfalls wenigstens ein weiteres, von a) verschiedenes Tensid und
d) gegebenenfalls ein Neutralsalz.

Bei den Polyglycosiden handelt es sich vorzugsweise um Polyglucoside, die ein durch Acetalisierung von Glucose mit Fettalkoholen erhaltenes Homologengemisch darstellen. Die mittlere Anzahl an Glucoseeinheiten pro Molekül liegt im Bereich von 1 bis 3.

Das Amid der Formel I ist in den Zusammensetzungen im Allgemeinen in einer Menge von mindestens 0,1 Gew.-%, vorzugsweise in einer Menge von 0,5 bis 20 Gew.-%, bezogen auf das Gesamtgewicht des Mittels, enthalten.

Als Tensid können in den erfindungsgemäßen Mitteln anionische, nichtionische, kationische und amphotere Tenside enthalten sein. Es hat sich gezeigt, dass die Amide der Formel I auch mit anionischen Tensiden verträglich sind.

Beispiele für Tenside sind Alkylpolyglycoside, Fettalkoholsulfate, Fettalkoholsulfonate, Fettalkoholethersulfate, Fettalkoholethersulfonate, Alkansulfonate, Fettalkoholethoxilate, Fettalkoholphosphate, Alkylbetaine, Sorbitanester, POE-Sorbitanester, Zuckerfettsäureester, Fettsäurepolyglycerinester, Fettsäurepartialglyceride, Fettsäurecarboxylate, Fettalkoholsulfosuccinate, Fettsäuresarcosinate, Fettsäureisethionate, Fettsäuretaurinate, Zitronensäureester, Silikon-Copolymere, Fettsäurepolyglykolester, Fettsäureamide, Fettsäurealkanolamide, quartäre Ammoniumverbindungen, Alkylphenoloxethylate, Fettaminoxethylate.

Vorzugsweise kommt in den erfindungsgemäßen Zusammensetzungen ein anionisches Tensid und/oder ein nichtionisches Tensid zur Anwendung, wobei als anionisches Tensid besonders bevorzugt ein Fettalkoholsulfat, Fettalkoholsulfonat, Fettalkoholethersulfonat, Alkansulfonat und insbesondere ein Fettalkoholethersulfat und als nichtionisches Tensid ein Alkyl- oder Alkenylpolyglycosid verwendet wird.

Eine weitere bevorzugte Ausführungsform ist eine wässrige Zusammensetzung in Form einer gießbaren Lösung, die wenigstens ein Amid der Formel I und wenigstens ein C₈-C₁₆-Alkyl- oder C₈-C₁₆-Alkenylpolyglycosid enthält. Diese Zusammensetzung enthält im Allgemeinen 10 bis 95 Gew.-%, vorzugsweise 60 bis 90 Gew.-% des Amids, bezogen auf die Gesamtmenge der beiden Komponenten. Die Gesamtmenge der Komponenten in der Zusammensetzung beträgt im Allgemeinen 30 bis 95 Gew.-%.

Zur zusätzlichen Verdickung können die erfindungsgemäßen Zusammensetzungen ein Neutralsalz, insbesondere Natriumsulfat und vorzugsweise Natriumchlorid, enthalten. Das Neutralsalz ist im Allgemeinen in einer Menge von 0,1 bis 10 Gew.-%, insbesondere 0,5 bis 10 Gew.-%, enthalten.

Darüber hinaus können die erfindungsgemäßen Zusammensetzungen übliche, dem Fachmann bekannte Hilfs- und Zusatzstoffe enthalten, beispielsweise Cosolventien wie Ethylenglykol, Propylenglykol, Glycerin, Lanolin-Derivate, Chloesterin-Derivate, Isopropylmyristat, Isopropylpalmitat, Elektrolyte, Farbstoffe, Konservierungsmittel, Säuren (beispielsweise Milchsäure, Citronensäure) etc.

Jeweils bezogen auf das Gesamtgewicht der Inhaltsstoffe (ausgenommen Wasser) enthalten die erfindungsgemäßen Mittel im Allgemeinen:
- 0,5 bis 50 Gew.-%, vorzugsweise 1 bis 25 Gew.-% wenigstens eines Amids der Formel I,
- 50 bis 99,5 Gew.-%, vorzugsweise 65 bis 90 Gew.-% wenigstens eines Tensids und
- 0 bis 50 Gew.-%, vorzugsweise 0,5 bis 30 Gew.-% wenigstens eines Neutralsalzes,
wobei sich die Mengen zu 100 Gew.-% addieren.

Gemäß einer bevorzugten Ausführungsform enthalten die erfindungsgemäßen Zusammensetzungen (bezogen auf das Gesamtgewicht der Inhaltsstoffe):
a) 10 bis 90 Gew.-%, vorzugsweise 15 bis 85 Gew.-% wenigstens eines C₈-C₁₆-Alkyl- oder C₈-C₁₆-Alkenylpolyglycosids;
b) 0,5 bis 50 Gew.-%, vorzugsweise 1 bis 25 Gew.-% wenigstens eines Amids der Formel I;
c) 10 bis 90 Gew.-%, vorzugsweise 15 bis 85 Gew.-% wenigstens eines anionischen Tensids, insbesondere eines Alkylethersulfates, und
d) 0 bis 50 Gew.-%, vorzugsweise 0,5 bis 30 Gew.-% wenigstens eines Neutralsalzes,
wobei sich die Mengen zu 100 Gew.-% addieren.

Die Herstellung dieser erfindungsgemäßen Zusammensetzungen erfolgt in üblicher Weise, wobei die Amide der polymerisierten Fettsäuren als solche oder als wässrige Lösung eingesetzt werden können. Im Allgemeinen wird das Verdickungsmittel in die wässrige Zusammensetzung eingerührt.

Bei den erfindungsgemäßen Zusammensetzungen handelt es sich insbesondere um kosmetische (Shampoos), pharmazeutische oder dietätische Zusammensetzungen. Die Amide der polymerisierten Fettsäuren können jedoch auch in technischen Präparaten eingesetzt werden, wie Hydraulikflüssigkeiten, Reinigungspräparaten, Pflanzenbehandlungsmitteln, Druckfarben, Anstrichmittel und Präparate für die Tierernährung.

Die nachfolgenden Beispiele erläutern die Erfindung, ohne sie zu begrenzen.

Die in den Beispielen gebrauchten Abkürzung haben folgende Bedeutungen:
SZ: Säurezahl
OHZ: Hydroxylzahl

### Beispiele

### Beispiel 1

115,0 g Pripol 1025 (polymerisierte Fettsäure, Fa. Unichema; SZ = 194 mg KOH/g) wurden bei 80 °C mit 30,8 g 1-Amino-2-propanol und 0,1 g Kaliumcarbonat versetzt und 12 Stunden bei 155 bis 160 °C unter N₂-Schutzgas gerührt. Das sich bildende Reaktionswasser wurde abdestilliert. Man erhielt 132 g des entsprechenden Amids.
SZ = 1,5 mg KOH/g
OHZ = 149 mg KOH/g

### Beispiel 2

85,2 g Pripol 1009 (polymerisierte Fettsäure, Fa. Unichema; SZ = 193 mg KOH/g) wurden bei 80 °C mit 18,3 g Monoethanolamin und 0,1 g Kaliumcarbonat versetzt und 6 Stunden bei 150 bis 155 °C unter N₂-Schutzgas gerührt. Das sich bildende Reaktionswasser wurde abdestilliert. Man erhielt 96 g des entsprechenden Amids.
SZ = 6,6 mg KOH/g
OHZ = 160 mg KOH/g

### Beispiel 3

54,7 g Aminosorbit wurden bei 150 °C mit 85,8 g Pripol 1098 (polymerisierte Fettsäure, Fa. Unichema; SZ = 198 mg KOH/g) und 0,14 g Kaliumcarbonat versetzt und 6 Stunden bei 150 °C unter N₂-Schutzgas gerührt. Das sich bildende Reaktionswasser wurde abdestilliert. Man erhielt 124 g des entsprechenden Amids.
SZ = 8,2 mg KOH/g
OHZ = 583 mg KOH/g

### Beispiel 4

54,7 g Aminosorbit wurden bei 150 °C mit 87,2 g Pripol 1009 (polymerisierte Fettsäure, Fa. Unichema; SZ = 193 mg KOH/g) und 2,84 g 50%iger hypophosphoriger Säure versetzt und 10 Stunden bei 150 °C unter N₂-Schutzgas gerührt. Das sich bildende Reaktionswasser wurde abdestilliert. Man erhielt 120 g des entsprechenden Amids.
SZ = 14,0 mg KOH/g
OHZ = 506 mg KOH/g

### Beispiel 5

39,0 g N-Methylglucamin wurden bei 150 °C mit 58,1 g Pripol 1009 (polymerisierte Fettsäure, Fa. Unichema; SZ = 193 mg KOH/g) und 0,10 g Kaliumcarbonat versetzt und 7 Stunden bei 150 °C unter N₂-Schutzgas gerührt. Das sich bildende Reaktionswasser wurde abdestilliert. Man erhielt 79 g des entsprechenden Amids.
SZ = 5,8 mg KOH/g
OHZ = 448 mg KOH/g

### Beispiel 6

Es wurde folgende Zusammensetzung hergestellt:

| | |
|---|---|
| Plantaren 2000 (C₈-C₁₆-Alkylpolyglycosid; 50 %-ig) | 160 g |
| Texapon NSO (Natriumlaurylethersulfat; 28 %-ig) | 220 g |
| vollentsalztes Wasser | 610 g |

Der pH-Wert der Zusammensetzung wurde durch Zugabe von Citronensäure auf 6,0 eingestellt. Anschließend wurden 3 Gew.-% Natriumchlorid, bezogen auf das Gesamtgewicht der Zusammensetzung, zugegeben. Die erhaltene Zusammensetzung hatte dann eine Viskosität von 12,5 mPas. In gleiche Teile dieser Zusammensetzung wurden anschließend jeweils 2 Gew.-%, 2,5 Gew.-% und 3 Gew.-% des gemäß Beispiel 1 oder gemäß Beispiel 2 erhaltenen Amides eingerührt und die Viskosität bestimmt. Die Viskositätsmessungen erfolgten mit einem Gerät der Bezeichnung HAAKE VT 500, Messeinrichtung PK5-1°; Temperatur 20 °C; Schergeschwindigkeit 30 s⁻¹. Die erhaltenen Viskositäten (in mPas) sind in der nachfolgenden Tabelle zusammengestellt.

| Amid | 0 % | 2,5 % | 3 % |
|---|---|---|---|
| Beispiel 1 | 12,5 | 1500 | 1800 |
| Beispiel 2 | 12,5 | 2150 | - |
| Beispiel 3 | 12,5 | - | 4400 |
| Beispiel 4 | 12,5 | - | 3750 |
| Beispiel 5 | 12,5 | - | 2750 |

## Patentansprüche

1. Verwendung von Amiden polymerisierter Fettsäuren der Formel I 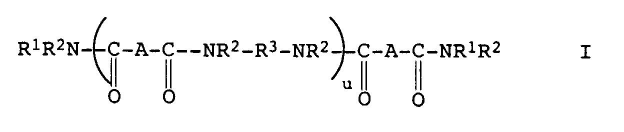 worin die Variablen unabhängig voneinander folgende Bedeutung haben:
A Rest einer dimerisierten Fettsäure mit 12 bis 110 C-Atomen;
R¹ C₁-C₁₂-Alkyl- oder C₃-C₈-Cycloalkylrest, der gegebenenfalls 1 bis 6 Substituenten aufweisen kann, die unabhängig voneinander ausgewählt sind unter Hydroxy, C₁-C₄₋Alkoxy, das gegebenenfalls durch 1 oder 2 Hydroxy- oder C₁-C₄₋Alkoxygruppen substituiert sein kann, Amino, C₁-C₄-Monoalkylamino und Di-C₁-C₄-alkylamino, wobei die Alkylgruppen der Aminogruppen unabhängig voneinander durch 1 oder 2 Hydroxygruppen substituiert sein können;
R² H oder C₁-C₄-Alkyl, das durch 1 oder 2 Hydroxygruppen substituiert sein kann;
R³ C₂-C₁₂-Alkylen, das 1 bis 6 Substituenten aufweisen kann, die unabhängig voneinander ausgewählt sind unter Hydroxy, C₁-C₄-Alkoxy, das durch 1 oder 2 Hydroxygruppen substituiert sein kann, Amino, C₁-C₄-Monoalkylamino und Di-C₁-C₄-alkylamino, wobei die Alkylgruppen der Aminogruppen durch 1 oder 2 Hydroxygruppen substituiert sein können;
u 0 bis 20;
als Verdickungsmittel.

2. Verwendung von Amiden polymerisierter Fettsäuren der Formel I nach Anspruch 1, wobei A für den Rest einer dimerisierten Fettsäure mit 22 bis 42 C-Atomen, vorzugsweise 30 bis 38 C-Atomen, steht.

3. Verwendung von Amiden polymerisierter Fettsäuren der Formel I nach Anspruch 1 oder 2, wobei R¹ für einen C₁-C₆-Alkylrest steht, der mit 1 bis 6 Hydroxy- und/oder Aminogruppen substituiert ist.

4. Verwendung von Amiden polymerisierter Fettsäuren der Formel I nach einem der vorhergehenden Ansprüche als Verdickungsmittel in kosmetischen Zusammensetzungen.

5. Wässrige Zusammensetzung, enthaltend wenigstens ein Amid einer polymerisierten Fettsäure der Formel I, wie in einem der Ansprüche 1 bis 3 definiert, und wenigstens ein Tensid, ausgenommen ein Betaintensid der Formel 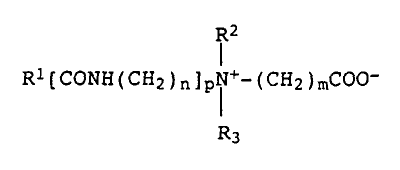 in der R¹ für einen aliphatischen Alkylrest mit 8 bis 22 C-Atomen, R² und R³ unabhängig voneinander für einen Alkyl- und/oder Hydroxyalkylrest mit 1 bis 4 C-Atomen, n und m unabhängig voneinander für Zahlen im Bereich von 1 bis 5 und p für 0 oder 1 steht.

6. Wässrige Zusammensetzung, enthaltend:
a) wenigstens ein Alkyl- oder Alkenylpolyglycosid,
b) wenigstens ein Amid einer polymerisierten Fettsäure der Formel I wie in einem der Ansprüche 1 bis 3 definiert,
c) gegebenenfalls wenigstens ein weiteres Tensid, und
d) gegebenenfalls wenigstens ein Neutralsalz.

7. Zusammensetzung nach Anspruch 5 oder 6, enthaltend 0,5 bis 20 Gew.-% des Amides der Formel I.

8. Zusammensetzung nach Anspruch 6, enthaltend:
a) 10 bis 90 Gew.-% eines Alkyl- oder Alkenylpolyglycosids,
b) 0,5 bis 50 Gew.-% eines Amids der Formel I,
c) 10 bis 90 Gew.-% eines anionischen Tensides, und
d) 0 bis 50 Gew.-% eines Neutralsalzes,
bezogen auf das Gesamtgewicht der Komponenten.

9. Zusammensetzung nach einem der Ansprüche 5 bis 8 in Form eines kosmetischen Mittels.

10. Zusammensetzung nach Anspruch 6, enthaltend
a) 10 bis 95 Gew.-% eines Amids der Formel I und
b) 5 bis 90 Gew.-% eines Alkyl- oder Alkenylpolyglycosids,
bezogen auf das Gesamtgewicht der beiden Komponenten.

11. Verfahren zur Erhöhung der Viskosität von tensidhaltigen Zusammensetzungen, wobei man der Zusammensetzung eine verdickend wirkende Menge wenigstens eines Amides polymerisierter Fettsäuren der Formel I, wie in einem der Ansprüche 1 bis 3 definiert, zusetzt.

12. Amide polymerisierter Fettsäuren der Formel I 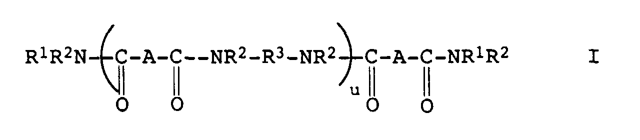 worin die Variablen unabhängig voneinander folgende Bedeutung haben:
A Rest einer dimerisierten Fettsäure mit 12 bis 110 C-Atomen;
R¹ Rest eines Aminozuckers;
R² H oder C₁-C₄-Alkyl, das durch 1 oder 2 Hydroxygruppen substituiert sein kann;
R³ C₂-C₁₂-Alkylen, das 1 bis 6 Substituenten aufweisen kann, die unabhängig voneinander ausgewählt sind unter Hydroxy, C₁-C₄-Alkoxy, das durch 1 oder 2 Hydroxygruppen substituiert sein kann, Amino, C₁-C₄-Monoalkylamino und Di-C₁-C₄-alkylamino, wobei die Alkylgruppen der Aminogruppen durch 1 oder 2 Hydroxygruppen substituiert sein können.

13. Amide polymerisierter Fettsäuren nach Anspruch 11 der Formel I, worin R¹ für einen Aminosorbit-, Glucosamin- oder Galactosaminrest steht.
